# EUROPEAN PATENT APPLICATION

(11) **EP 3 686 155 A1**
(43) Date of publication of application: **29.07.2020**
(21) Application number: 18858647.3
(22) Date of filing: 28.08.2018
(51) Int. Cl.: C01B 11/02, A61L 9/01, A61L 9/015

(54) **METHOD FOR GENERATING CHLORINE DIOXIDE GAS, LIQUID COMPOSITION, GEL COMPOSITION, AND CHLORINE DIOXIDE GAS GENERATING KIT**

(30) Priority: 20.09.2017 JP 2017180688; 02.07.2018 JP 2018126230
(71) Applicant: CLO2 Lab Inc., Nishinomiya-shi, Hyogo 662-0017 (JP)
(72) Inventor: ABE Koji, Nishinomiya-shi Hyogo 662-0017 (JP); ABE Tsukasa, Nishinomiya-shi Hyogo 662-0017 (JP)
(74) Representative: 2SPL Patentanwälte PartG mbB
(86) International application number: PCT/JP2018/031726
(87) International publication number: WO 2019/058892

(57) **Abstract**

Chlorine dioxide gas is immediately generated at a stable concentration from a liquid composition. The composition is obtained by mixing an aqueous chlorite solution, an activator that immediately adjusts the pH of the aqueous chlorite solution, an activation inhibitor that slowly mitigates an action of the activator, and a metal iodide such that the metal iodide is contained in a proportion of 0.4% by mass or less and the activation inhibitor is contained in a proportion of 1% by mass or less with respect to 1 L of 1% by mass aqueous chlorite solution.

## Description

### Technical Field

The present invention relates to a technique for immediately generating chlorine dioxide gas at a stable concentration.

### Background Art

It is known that chlorine dioxide has strong oxidizability, and kills bacteria or degrades offensive odor components through its oxidizing action. Accordingly, chlorine dioxide is widely used as an antimicrobial agent, a deodorant, a fungicide, bleach, and the like. In these applications, chlorine dioxide is often used in the form of chlorine dioxide gas.

As an example of a chlorine dioxide gas generating method, a method in which an activator such as an organic acid or an inorganic acid is added to an aqueous chlorite solution is disclosed, for example, in JP 2005-29430A (Patent Document 1). In the method disclosed in Patent Document 1, the amount of chlorine dioxide gas generated is adjusted using a gas generation adjuster such as sepiolite or zeolite. Although not specifically described in Patent Document 1, it is assumed that, since sepiolite and zeolite are porous materials, the amount of gas generated is adjusted by retaining excessive gas in the gas generation adjuster when the amount of gas generated is large, and releasing the retained gas when the amount of gas generated is small.

However, it is not possible to sufficiently adjust the amount of gas generated merely through the physical adsorbing action, and it is not possible to sufficiently suppress an abrupt increase in the chlorine dioxide gas concentration after an activator is added to the aqueous chlorite solution. Accordingly, although Patent Document 1 states that chlorine dioxide gas is continuously generated, it will be appreciated that the effect is limited. If a weak acid is used as the activator, an abrupt increase in the chlorine dioxide gas concentration can be suppressed, but there is a problem in that the effect cannot be immediately achieved because it requires time to reach the maximum concentration. Furthermore, the concentration of generated chlorine dioxide gas depends only on the concentration of chlorite, and control of the maximum concentration is not possible.

### Prior Art Document

### Patent Document

Patent Document 1: JP 2005-29430A

### Disclosure of the Invention

### Problem to be Solved by the Invention

There is demand for being able to freely control the concentration of generated chlorine dioxide gas and generate chlorine dioxide gas immediately and stably for a long period of time.

### Means for Solving Problem

The present invention is directed to a first chlorine dioxide gas generating method for immediately generating chlorine dioxide gas at a stable concentration from a liquid composition, including obtaining the composition by mixing an aqueous chlorite solution, an activator that immediately adjusts a pH of the aqueous chlorite solution, thereby causing the aqueous chlorite solution to generate chlorine dioxide gas, an activation inhibitor that slowly mitigates an action of the activator, and a metal iodide such that the metal iodide is contained in a proportion of 0.4% by mass or less and the activation inhibitor is contained in a proportion of 1% by mass or less with respect to 1 L of 1% by mass aqueous chlorite solution.

The present invention is directed to a second chlorine dioxide gas generating method for immediately generating chlorine dioxide gas at a stable concentration from a gel composition, including obtaining the composition by mixing an aqueous chlorite solution, an activator that immediately adjusts a pH of the aqueous chlorite solution, thereby causing the aqueous chlorite solution to generate chlorine dioxide gas, an activation inhibitor that slowly mitigates an action of the activator, a metal iodide, and an absorbent resin such that the metal iodide is contained in a proportion of 0.4% by mass or less and the activation inhibitor is contained in a proportion of 1% by mass or less with respect to 1 L of 1% by mass aqueous chlorite solution.

The present invention is directed to a liquid composition for immediately generating chlorine dioxide gas at a stable concentration, including an aqueous chlorite solution, an activator that immediately adjusts a pH of the aqueous chlorite solution, thereby causing the aqueous chlorite solution to generate chlorine dioxide gas, an activation inhibitor that slowly mitigates an action of the activator, and a metal iodide such that the metal iodide is contained in a proportion of 0.4% by mass or less and the activation inhibitor is contained in a proportion of 1% by mass or less with respect to 1 L of 1% by mass aqueous chlorite solution.

The present invention is directed to a gel composition for immediately generating chlorine dioxide gas at a stable concentration, including an aqueous chlorite solution, an activator that immediately adjusts a pH of the aqueous chlorite solution, thereby causing the aqueous chlorite solution to generate chlorine dioxide gas, an activation inhibitor that slowly mitigates an action of the activator, a metal iodide, and an absorbent resin such that the metal iodide is contained in a proportion of 0.4% by mass or less and the activation inhibitor is contained in a proportion of 1% by mass or less with respect to 1 L of 1% by mass aqueous chlorite solution.

The present invention is directed to a first chlorine dioxide gas generating kit for immediately generating chlorine dioxide gas at a stable concentration from a liquid composition, including:
a first agent containing an aqueous chlorite solution; and
a second agent containing an activator that immediately adjusts a pH of the aqueous chlorite solution, thereby causing the aqueous chlorite solution to generate chlorine dioxide gas,
wherein each of an activation inhibitor that slowly mitigates an action of the activator, and a metal iodide is contained in either the first agent or the second agent,
0.4% by mass or less of the metal iodide and 1% by mass or less of the activation inhibitor are contained with respect to 1 L of 1% by mass aqueous chlorite solution, and
the composition is obtained by mixing the first agent and the second agent.

The present invention is directed to a second chlorine dioxide gas generating kit for immediately generating chlorine dioxide gas at a stable concentration from a gel composition, including:
a first agent containing an aqueous chlorite solution; and
a second agent containing an activator that immediately adjusts a pH of the aqueous chlorite solution, thereby causing the aqueous chlorite solution to generate chlorine dioxide gas, and an absorbent resin,
wherein each of an activation inhibitor that slowly mitigates an action of the activator, and a metal iodide is contained in either the first agent or the second agent,
0.4% by mass or less of the metal iodide and 1% by mass or less of the activation inhibitor are contained with respect to 1 L of 1% by mass aqueous chlorite solution, and
the composition is obtained by mixing the first agent and the second agent.

With these configurations, when the components are mixed, the activator immediately acts, thereby causing chlorine dioxide gas to be immediately generated. At this time, 0.4% by mass or less of metal iodide acts as a catalyst, thereby facilitating the generation of chlorine dioxide gas in the early stage of mixing. Thus, for example, even when a weak acid is used as the activator, the time taken until chlorine dioxide gas reaches the maximum concentration can be shortened. Subsequently, 1% by mass or less of activation inhibitor slowly acts, thereby mitigating the action of the activator, and slowing down the generation of chlorine dioxide gas. Accordingly, while chlorine dioxide gas is immediately generated in the early stage after mixing, an abrupt increase in the concentration is inhibited, and chlorine dioxide gas will be gradually released. Accordingly, it is possible to generate chlorine dioxide gas immediately and stably for a long period of time. Furthermore, it is possible to freely control the concentration of generated chlorine dioxide gas by adjusting the amount of activation inhibitor added.

Hereinafter, preferred embodiments of the present invention will be described. Note that the scope of the present invention is not limited to the preferred embodiment examples described below.

In an aspect, it is preferable that 0.03% by mass or more and 0.3% by mass or less of the activation inhibitor is contained with respect to 1 L of 1% by mass aqueous chlorite solution.

In an aspect, it is preferable that 0.01% by mass or more and 0.4% by mass or less of the metal iodide is contained with respect to 1 L of 1% by mass aqueous chlorite solution.

In an aspect, it is preferable that a mass ratio between the activation inhibitor and the metal iodide is 3 : 1 to 1 : 3.

With these configurations, it is possible to freely control the concentration of generated chlorine dioxide gas, and to generate chlorine dioxide gas immediately and stably for a long period of time, in a particularly satisfactory manner.

In an aspect, it is preferable that the activation inhibitor is an alkali metal silicate or an alkaline-earth metal silicate.

With this configuration, when an alkali metal silicate or an alkaline-earth metal silicate is dissolved in an aqueous solution, hydroxide ions can be produced through hydrolysis. Thus, it is possible to slowly mitigate the action of an activator, which is typically an acid, through a neutralization reaction, and to freely control the concentration of chlorine dioxide gas.

In an aspect, it is preferable that the activation inhibitor is a sodium silicate.

With this configuration, it is possible to freely control the concentration of chlorine dioxide gas, at low cost, using a sodium silicate that is easily available and relatively inexpensive.

In an aspect, it is preferable that the metal iodide is a potassium iodide.

With this configuration, it is possible to facilitate the generation of chlorine dioxide gas in the early stage of mixing, at low cost, using a potassium iodide that is easily available and relatively inexpensive.

In an aspect, it is preferable that the activator is an inorganic acid or an organic acid, or a salt thereof.

With this configuration, it is possible to promptly and appropriately generate chlorine dioxide gas in the early stage after mixing the components.

In an aspect, it is preferable that the first agent is sealed in a sealable first container, and the second agent is sealed in a sealable second container that is different from the first container.

With this configuration, it is possible to prevent oxygen or moisture in air from being mixed in, and to prevent the first agent and the second agent from deteriorating. Thus, it is possible to stably store the first agent and the second agent for a long period of time before use.

In an aspect, it is preferable that the first agent and the second agent are sealed in the same sealable container in a state of being separated from each other by a separating portion that can be manually deactivated.

With this configuration, it is possible to prevent oxygen or moisture in air from being mixed in, and to prevent the first agent and the second agent from deteriorating. Thus, it is possible to stably store the first agent and the second agent for a long period of time before use. Furthermore, with this configuration, it is possible to collectively handle the first agent and the second agent in the same sealable container, and the portability is excellent. At the time of use, the first agent and the second agent can be mixed with ease in the same sealable container, merely by manually deactivating the separating portion.

In an aspect, it is preferable that the separating portion is constituted by a labyrinth structure portion.

With this configuration, it is possible to more reliably prevent the aqueous chlorite solution contained in the first agent from permeating the separating portion and reaching the second agent during storage. Thus, it is possible to more reliably prevent chlorine dioxide gas from accidentally being generated during storage.

In an aspect, it is preferable that the first agent is sealed in a sealable and easily breakable first container, and the second agent is sealed together with the first container in a sealable second container.

With this configuration, it is possible to prevent oxygen or moisture in air from being mixed in, and to prevent the first agent and the second agent from deteriorating. Thus, it is possible to stably store the first agent and the second agent for a long period of time before use. Furthermore, with this configuration, it is possible to collectively handle the first agent sealed in the first container together with the second agent in the second container, and the portability is excellent. At the time of use, the first agent and the second agent can be mixed with ease in the second container, merely by breaking the first container by applying a force from the outside.

Further features and advantages of the technique according to the present invention will become apparent from the following description of illustrative and non-limiting embodiments with reference to the drawings.

### Brief Description of the Drawings

FIG. 1 is a diagram illustrating the principle of a generating method for gradually releasing chlorine dioxide gas.
FIG. 2 is a graph showing the chlorine dioxide gas concentration over time.
FIG. 3 is a schematic view showing the appearance of a chlorine dioxide gas generating kit according to a first embodiment.
FIG. 4 is a schematic view showing an aspect of a chlorine dioxide gas generating method.
FIG. 5 is a schematic view showing an example of a use mode of a gel composition.
FIG. 6 is a schematic view showing the appearance of a chlorine dioxide gas generating kit according to a second embodiment.
FIG. 7 is a cross-sectional view enlarging a portion near a separating portion.
FIG. 8 is a schematic view showing an aspect of a chlorine dioxide gas generating method.
FIG. 9 is a schematic view showing an example of a use mode of a gel composition.
FIG. 10 is a schematic view showing the appearance of a chlorine dioxide gas generating kit according to a third embodiment.

### Best Mode for Carrying out the Invention

### First Embodiment

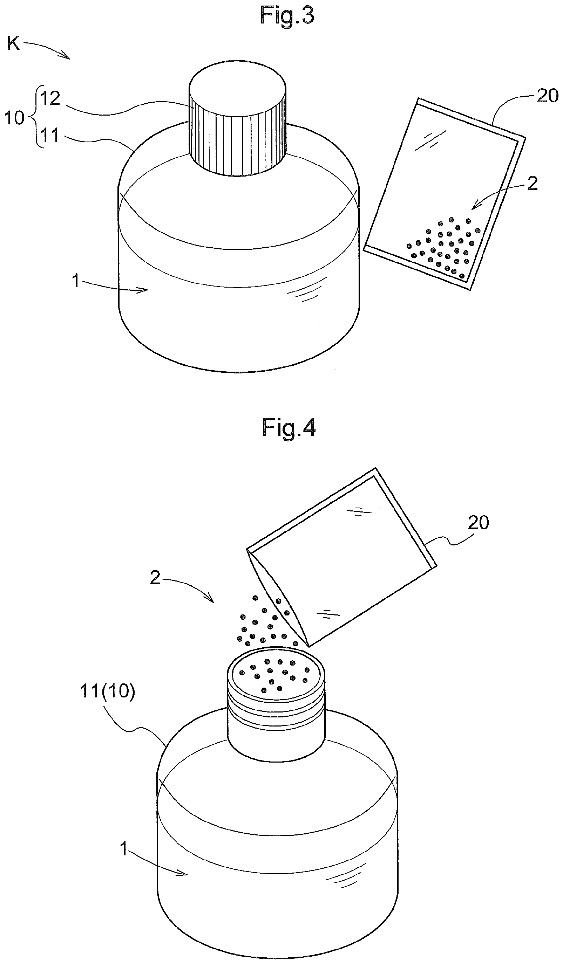

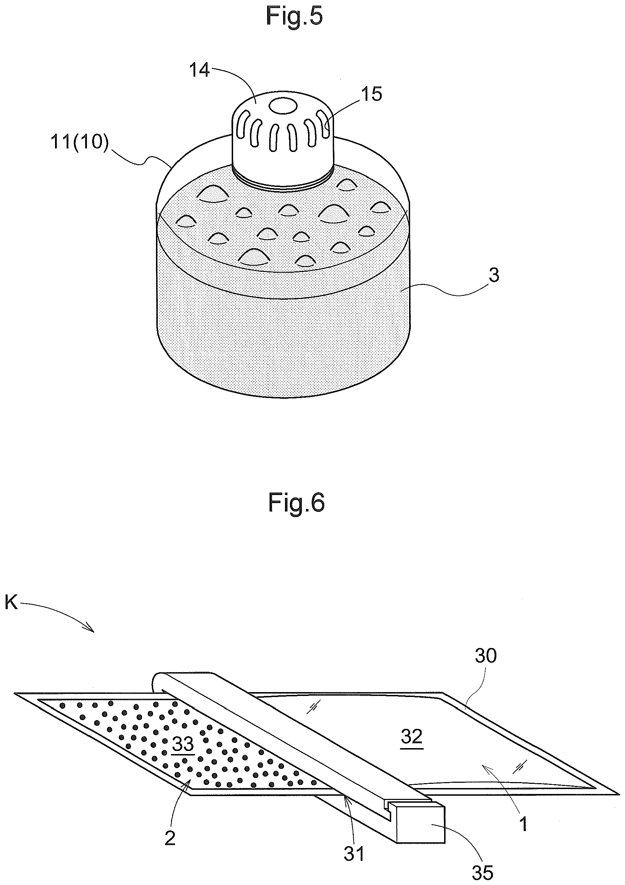

Hereinafter, a chlorine dioxide gas generating method, a liquid composition, a gel composition, and a chlorine dioxide gas generating kit according to a first embodiment will be described. The chlorine dioxide gas generating method of this embodiment is a method for immediately generating chlorine dioxide gas at a stable concentration, by mixing an aqueous chlorite solution, a fast-acting activator, a metal iodide, a slow-acting activation inhibitor, and, optionally, an absorbent resin. In this embodiment, this method is performed using a chlorine dioxide gas generating kit K (see FIG. 3) including a first agent 1 containing an aqueous chlorite solution, a metal iodide, and a slow-acting activation inhibitor, and a second agent 2 containing a fast-acting activator, and, optionally, an absorbent resin. It is possible to immediately generate chlorine dioxide gas at a stable concentration, from a liquid composition or a gel composition 3 (see FIG. 5) obtained by mixing the first agent 1 and the second agent 2 of the chlorine dioxide gas generating kit K.

In the description below, as an example, a case will be described in which chlorine dioxide gas is immediately generated at a stable concentration from the gel composition 3 by also mixing the absorbent resin that is an optional component.

The aqueous chlorite solution is an aqueous solution containing chlorite. There is no particular limitation on the chlorite contained in the aqueous chlorite solution, as long as it is substantially stable, and is activated by being mixed with the activator and produces chlorine dioxide gas. Examples of the chlorite include alkali metal chlorite and alkaline-earth metal chlorite. Examples of the alkali metal chlorite include sodium chlorite (NaClO₂), potassium chlorite (KClO₂), and lithium chlorite (LiClO₂). Examples of the alkaline-earth metal chlorite include calcium chlorite (Ca (ClO₂)₂), magnesium chlorite (Mg (ClO₂)₂), and barium chlorite (Ba (ClO₂)₂). Of these, it is preferable to use sodium chlorite.

There is no particular limitation on the pH of the aqueous chlorite solution before mixing, but it is preferably 9 or more and 13 or less. The pH of the aqueous chlorite solution is more preferably 10 or more and 12.5 or less, and even more preferably 11 or more and 12 or less. If the pH is within this range, the chlorite in the aqueous chlorite solution can be stabilized and stably stored for a long period of time. The pH of the aqueous chlorite solution can be adjusted using an alkali agent. Examples of the alkali agent include sodium hydroxide (NaOH) and potassium hydroxide (KOH).

The chlorite concentration of the aqueous chlorite solution is preferably 0.01% by mass or more and 25% by mass or less, and more preferably 0.1% by mass or more and 15% by mass or less.

The activator activates the chlorite in the aqueous chlorite solution, when mixed with the solution, thereby causing the chlorite to generate chlorine dioxide gas. Examples of the activator include an inorganic acid and an organic acid, and a salt thereof. Examples of the inorganic acid include hydrochloric acid (HCl), carbonic acid (H₂CO₃), sulfuric acid (H₂SO₄), phosphoric acid (H₃PO₄), and boric acid (H₃BO₃). Examples of a salt of the inorganic acid include sodium hydrogen carbonate (NaHCO₃), sodium dihydrogen phosphate (NaH₂PO₄), and disodium hydrogen phosphate (Na₂HPO₄). As the inorganic acid and a salt thereof, it is also possible to use an anhydride (e.g., sulfuric anhydrite, pyrophosphoric acid, etc.), and, for example, it is preferable to use sodium dihydrogen pyrophosphate, or the like.

Examples of the organic acid include acetic acid (CH₃COOH), citric acid (H₃(C₃H₅O(COO)₃)), and malic acid (COOH(CHOH)CH₂COOH). Examples of a salt of the organic acid include sodium acetate (CH₃COONa), disodium citrate (Na₂H(C₃H₅O(COO)₃)), trisodium citrate (Na₃(C₃H₅O(COO)₃)), and disodium malate (COONa(CHOH)CH₂COONa).

The activator immediately adjusts the pH of the aqueous chlorite solution, when mixed with the aqueous chlorite solution. More specifically, the activator immediately lowers the pH of the aqueous chlorite solution, and provides an acidic atmosphere. In this sense, the activator can be said to be a "pH adjuster that immediately imparts acidity". The activator adjusts the pH of the aqueous chlorite solution preferably to 2.5 or more and 6.8 or less. The activator adjusts the pH of the aqueous chlorite solution more preferably to 3.5 or more and 6.5 or less, and even more preferably to 4.5 or more and 6.0 or less. Preferred examples of the activator include sodium metaphosphate whose 1% aqueous solution has a pH of 1.7 or more and 2.4 or less.

For example, if the chlorite contained in the aqueous chlorite solution is sodium chlorite, chlorous acid is produced following Formula (1) below, by adjusting the pH of the aqueous solution as described above to provide an acidic atmosphere.

NaClO₂ + H⁺ → Na⁺ + HClO₂ (1)

Meanwhile, the equilibrium reaction in a case in which chlorine dioxide gas is dissolved in water is expressed by Formula (2) below.

2ClO₂ + H₂O ⇔ HClO₂ + HClO₃ (2)

At that time, Formula (3) below is obtained.

[HClO₂][HClO₃]/[ClO₂] = 1.2 × 10⁻⁷ (3)

When chlorous acid is produced following Formula (1) by mixing the aqueous chlorite solution and the activator to set the aqueous chlorite solution to an acidic atmosphere, the equilibrium reaction shifts leftward in Formula (2) according to the theorem of Formula (3), and thus chlorine dioxide gas can be generated in the aqueous solution at an overwhelming probability.

In the chlorine dioxide gas generating method of this embodiment, in addition to the activator that immediately adjusts the pH of the aqueous chlorite solution (which will be referred to as a "first activator" in this example), a second activator that slowly adjusts the pH of the aqueous chlorite solution may be mixed as well. In this sense, the second activator can be said to be a "pH adjuster that slowly imparts acidity". The second activator may be an inorganic acid or organic acid with a level of acidity lower than that of the first activator, or a salt thereof. Preferred examples of the second activator include sodium pyrophosphate whose 1% aqueous solution has a pH of 3.8 or more and 4.5 or less.

The metal iodide produces iodide ions and acts as a catalyst in an aqueous chlorite solution, when mixed with the solution. The metal iodide facilitates the generation of chlorine dioxide gas in the early stage of mixing with the aqueous chlorite solution and the activator. Examples of the metal iodide include an alkali metal iodide and an alkaline-earth metal iodide. Specific examples thereof include a sodium iodide (NaI), a potassium iodide (KI), a magnesium iodide (MgI₂), and a calcium iodide (CaI₂). Of these, it is preferable to use a potassium iodide.

The amount of metal iodide added to the aqueous chlorite solution is 0.4% by mass or less, with respect to 1 L of 1% by mass aqueous chlorite solution (i.e., with respect to 10000 ppm of chlorite). There is no particular limitation on the lower limit value of the amount of metal iodide added, but it is set to an amount (* not including zero) that enables an effect of facilitating the generation of chlorine dioxide gas in the early stage of mixing to be obtained. The amount of metal iodide added is preferably 0.01% by mass or more and 0.4% by mass or less, with respect to 1 L of 1% by mass aqueous chlorite solution. The amount of metal iodide added is more preferably 0.1% by mass or more and 0.25% by mass or less, with respect to 1 L of 1% by mass aqueous chlorite solution.

The activation inhibitor slowly mitigates the action of the activator, when mixed with the aqueous chlorite solution together with the activator. The activation inhibitor slowly mitigates the action of the activator of immediately lowering the pH of the aqueous chlorite solution. The activation inhibitor may substantially be a material that slowly increases the pH of the aqueous chlorite solution. In this sense, the activation inhibitor can be said to be a "pH adjuster that slowly imparts alkalinity". Examples of the activation inhibitor include an alkali metal silicate and an alkaline-earth metal silicate. Examples of the alkali metal silicate include a lithium silicate (mLi₂O·nSiO₂), a sodium silicate (mNa₂O·nSiO₂), and a potassium silicate (mK₂O·nSiO₂). Examples of the alkaline-earth metal silicate include a magnesium silicate (mMgO·nSiO₂), a calcium silicate (mCaO·nSiO₂), and a strontium silicate (mSrO·nSiO₂). Of these, it is preferable to use a sodium silicate (in particular, a sodium metasilicate). It is assumed that the "alkali metal silicate" and the "alkaline-earth metal silicate" include hydrates thereof.

There is no particular limitation on the molar ratio (the above-mentioned n/m) between an oxide of an alkali metal or an alkaline-earth metal silicate and a silicon dioxide, but it is preferably 0.9 or more and 1.2 or less.

For example, if the activation inhibitor is a sodium metasilicate, the sodium metasilicate dissociates (hydrolyzes) in the aqueous solution as in Formula (4) below.

Na₂O·SiO₂ + 2H₂O → 2NaOH + H₂SiO₃ (4)

In this manner, sodium hydroxide (NaOH) produced after a short period of time has passed after mixing with the aqueous chlorite solution acts so as to partially neutralize the fast-acting activator (an acid in this example), thereby slowly mitigating the action of the activator. As a result, an abrupt increase in the chlorine dioxide gas concentration in the early stage after mixing is inhibited, and chlorine dioxide gas can be gradually released from the early stage.

Meanwhile, as in Formula (4), metasilicic acid (H₂SiO₃) is also produced in addition to sodium hydroxide. Metasilicic acid is produced after a short period of time has passed after mixing with the aqueous chlorite solution, and acts as an acid, and, in this sense, silicon dioxide (SiO₂) from which metasilicic acid is produced is an example of the "pH adjuster that slowly imparts acidity". Sodium hydroxide and metasilicic acid produced later further react with each other as in Formula (5) below.

2NaOH + H₂SiO₃ → Na₂O·SiO₂ + 2H₂O (5)

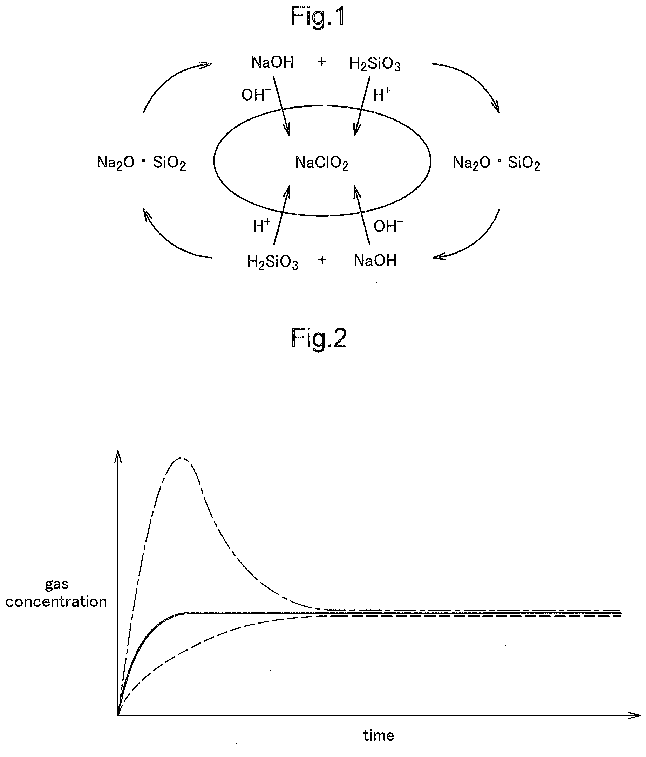

In this manner, sodium metasilicate serving as an activation inhibitor shifts between a state of being dissociated into sodium hydroxide and metasilicic acid and a state of being recombined, in the aqueous solution (see FIG. 1).

Then, sodium metasilicate in the state of being dissociated into sodium hydroxide and metasilicic acid slowly adjusts the pH of the aqueous chlorite solution. That is to say, in the state in which sodium metasilicate has dissociated into sodium hydroxide and metasilicic acid, metasilicic acid acts as a supply source of hydrogen ions (H⁺), and sodium hydroxide acts as a supply source of hydroxide ions (OH⁻), thereby slowly adjusting the pH of the aqueous chlorite solution. As a result, it is possible to slowly generate chlorine dioxide gas, and to generate chlorine dioxide gas at a stable concentration for a long period of time. Furthermore, when used together with the metal iodide, it is possible to immediately generate chlorine dioxide gas at a stable concentration.

In this example, "generated at a stable concentration" means that, in a closed system, the concentration of generated chlorine dioxide gas slowly increases without having a peak in the early stage after mixing and then keeps a constant level (see FIG. 2), or, even if there is a peak, the ratio of the peak concentration relative to the final concentration is kept sufficiently low. In the case of the latter, the ratio of the peak concentration relative to the final concentration is, for example, preferably 1.3 or less, more preferably 1.2 or less, and even more preferably 1.1 or less. Furthermore, "immediately generated at a stable concentration" means that, after mixing the aqueous chlorite solution and the activator, the concentration of generated chlorine dioxide gas abruptly increases and converges to the final concentration without having an apparent peak.

Note that, in FIG. 2, a change in the concentration of chlorine dioxide gas when the metal iodide and the activation inhibitor are mixed with the aqueous chlorite solution together with the activator in a closed system is indicated by the solid line. Furthermore, for the sake of comparison, a change in the concentration when only the activator is mixed without mixing the metal iodide and the activation inhibitor is indicated by the dash-dot line, and a change in the concentration when only the activator and the activation inhibitor are mixed without mixing the metal iodide is indicated by the broken line.

Furthermore, according to the method of this embodiment, it is possible to freely control the concentration of generated chlorine dioxide gas. Conventionally, the concentration of generated chlorine dioxide gas depends on the concentration of chlorite, and control of the maximum concentration was not possible, whereas, in this method, the maximum concentration (preferably, final concentration) of chlorine dioxide gas can be freely controlled by adjusting the amount of activation inhibitor added. Thus, it is possible to easily generate chlorine dioxide gas at a concentration suitable for the purpose of use.

The amount of activation inhibitor added to the aqueous chlorite solution is 1% by mass or less, with respect to 1 L of 1% by mass aqueous chlorite solution (i.e., with respect to 10000 ppm of chlorite). There is no particular limitation on the lower limit value of the amount of activation inhibitor added, but it is set to an amount (* not including zero) that enables an effect of realizing generation of chlorine dioxide gas at a stable concentration to be obtained. The amount of activation inhibitor added is preferably 0.03% by mass or more and 0.3% by mass or less, with respect to 1 L of 1% by mass aqueous chlorite solution. The amount of activation inhibitor added is more preferably 0.1% by mass or more and 0.25% by mass or less, with respect to 1 L of 1% by mass aqueous chlorite solution.

There is no particular limitation on the ratio between the amounts of metal iodide and activation inhibitor added, but it may be metal iodide: activation inhibitor = 3 : 1 to 1 : 3. The ratio between the amounts of metal iodide and activation inhibitor added is preferably 2 : 1 to 1 : 2, more preferably 1.5 : 1 to 1 : 1.5, and even more preferably 1.25 : 1 to 1 : 1.25.

The absorbent resin absorbs moisture, and forms a gel composition. Examples of the absorbent resin include a starch-based absorbent resin, a cellulose-based absorbent resin, and a synthetic polymer-based absorbent resin. Examples of the starch-based absorbent resin include a starch-acrylonitrile graft copolymer and a starch-acrylic acid graft copolymer. Examples of the cellulose-based absorbent resin include a cellulose-acrylonitrile graft copolymer and a cross-linked carboxymethylcellulose. Examples of the synthetic polymer-based absorbent resin include a polyvinyl alcohol-based absorbent resin and an acrylic-based absorbent resin.

The activator, the metal iodide, the activation inhibitor, and the absorbent resin may be a solid (e.g., in a powdery form or a granular form) before mixed with the aqueous chlorite solution. Furthermore, the activator, the metal iodide, and the activation inhibitor may be dissolved when mixed with the aqueous chlorite solution.

The chlorine dioxide gas generating method of this embodiment may be performed using the chlorine dioxide gas generating kit K shown in FIG. 3. The chlorine dioxide gas generating kit K includes a first agent 1 containing an aqueous chlorite solution, a metal iodide, and a slow-acting activation inhibitor, and a second agent 2 containing a fast-acting activator and an absorbent resin. In the chlorine dioxide gas generating kit K, the first agent 1 and the second agent 2 are respectively sealed in sealable containers. In this embodiment, the first agent 1 formed as a liquid (an aqueous chlorite solution in which the metal iodide and the activation inhibitor are dissolved) is contained in a first container 10 mainly constituted by a container main body 11 made of plastic. The first container 10 has a sealing cap 12, and, when the sealing cap 12 is attached to the container main body 11 in a liquid-tight manner, the first agent 1 is sealed in the sealable first container 10.

Furthermore, the second agent 2 formed as a solid (the activator and the absorbent resin) is contained in a second container 20 obtained by sticking plastic films to each other. The second container 20 may be obtained by stacking two plastic films and causing their entire peripheral edge portions to adhere to each other, or by folding one plastic film in half and causing the peripheral edge portions other than the folded portion to adhere to each other. In this manner, the second agent 2 is sealed in the sealable second container 20 that is different from the first container 10.

There is no limitation on the material and the shape of the first container 10 and the second container 20, as long as they are sealable containers. The material for forming the first container 10 and the second container 20 is not limited to plastic, and may be, for example, metal. Furthermore, the shape of the first container 10 is not limited to a fixed shape, and may be a deformable shape. The shape of the second container 20 is not limited to a deformable shape, and may be a fixed shape. Moreover, a configuration may also be employed in which the first agent 1 and the second agent 2 are contained in an integrated container having two container sections, and can be mixed with each other by bringing the two container sections into communication with each other at the time of use.

In the chlorine dioxide gas generating kit K of this embodiment, the first agent 1 is distributed in the form of an aqueous chlorite solution, and the storage safety is excellent. For example, the storage safety is higher than that in a case of distributing an aqueous chlorite solution in which chlorine dioxide gas is dissolved while keeping the pH acidic.

Chlorine dioxide gas can be actually generated using the chlorine dioxide gas generating kit K of this embodiment as follows. That is to say, as shown in FIG. 4, the sealing cap 12 is detached from the container main body 11 of the first container 10 containing the first agent 1. Furthermore, the second container 20 containing the second agent 2 is opened by cutting the plastic film. Then, when the second agent 2 in the second container 20 is inserted into the first container 10 (the container main body 11), the first agent 1 and the second agent 2 are mixed with each other. In this manner, the aqueous chlorite solution in which the metal iodide and the slow-acting activation inhibitor are dissolved, the fast-acting activator, and the absorbent resin are mixed with each other in the first container 10 (the container main body 11). That is to say, the aqueous chlorite solution, the fast-acting activator, the metal iodide, the slow-acting activation inhibitor, and the absorbent resin are mixed with each other as a whole in the first container 10 (the container main body 11).

Then, the content is converted into a gel form in the first container 10 (the container main body 11), and chlorine dioxide gas is immediately generated at a stable concentration from the obtained gel composition 3 (see FIG. 5). If an opening cap 14 having a plurality of openings 15 is attached to the container main body 11, generated chlorine dioxide gas is released via the openings 15 into a room. Thus, an antibacterial effect, a deodorant effect, and the like can be immediately provided at the time of use due to the strong oxidizability of immediately released chlorine dioxide gas, and an antibacterial effect, a deodorant effect, and the like can be stably provided for a long period of time by chlorine dioxide gas gradually released at a stable concentration.

In the description above, a configuration may also be employed in which the second agent 2 does not contain the absorbent resin, and only the aqueous chlorite solution, the fast-acting activator, the metal iodide, and the slow-acting activation inhibitor are mixed with each other. In this case, chlorine dioxide gas can be immediately generated at a stable concentration from the obtained liquid composition. Also, in this case, an antibacterial effect, a deodorant effect, and the like can be provided immediately at the time of use and stably for a long period of time due to the strong oxidizability of chlorine dioxide gas gradually released immediately and at a stable concentration.

### Second Embodiment

Hereinafter, a chlorine dioxide gas generating method, a liquid composition, a gel composition, and a chlorine dioxide gas generating kit according to a second embodiment will be described. In this embodiment, the aqueous chlorite solution, the fast-acting activator, the metal iodide, the slow-acting activation inhibitor, and the absorbent resin are allocated to the first agent 1 and the second agent 2 of the chlorine dioxide gas generating kit K in a manner different from that of the first embodiment. Furthermore, the specific configuration of the container of the chlorine dioxide gas generating kit K is different from that of the first embodiment.

Hereinafter, the chlorine dioxide gas generating kit K of this embodiment will be described mainly focusing on differences from the first embodiment. Note that constituent elements not specifically described are as in the first embodiment, and thus they are denoted by the same reference numerals, and a description thereof has been omitted.

The chlorine dioxide gas generating kit K of this embodiment includes a first agent 1 containing an aqueous chlorite solution, and a second agent 2 containing a fast-acting activator, a metal iodide, a slow-acting activation inhibitor, and an absorbent resin. As shown in FIG. 6, they are sealed in the same sealable container 30. The container 30 is made of a gas-permeable film. The container 30 may be obtained by stacking two gas-permeable films and causing their entire peripheral edge portions to adhere to each other, or by folding one gas-permeable film in half and causing the peripheral edge portions other than the folded portion to adhere to each other.

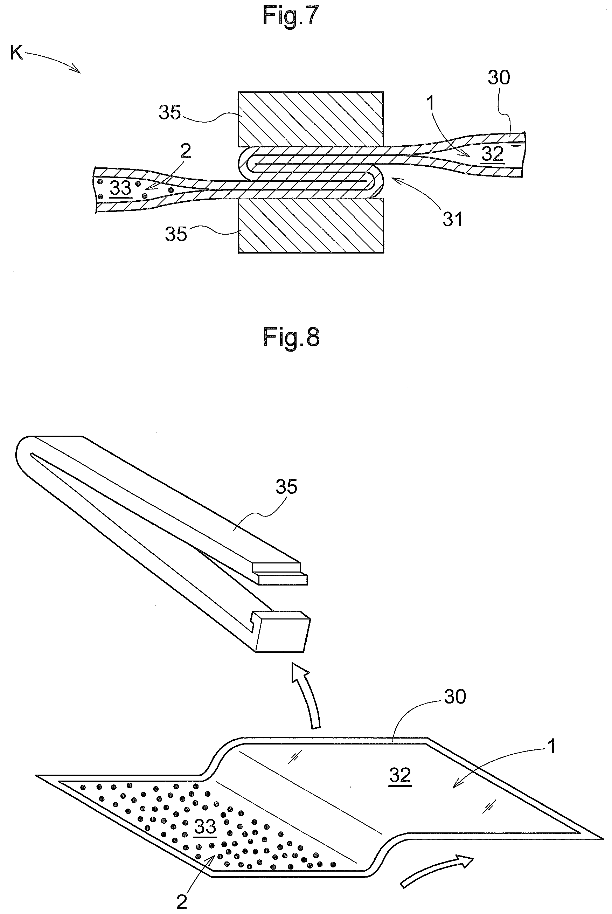

The internal portion of the container 30 is separated into two spaces consisting of a first container section 32 and a second container section 33 by a separating portion 31 that can be manually deactivated. As shown in FIG. 7, in this embodiment, the separating portion 31 is constituted by a labyrinth structure portion in which a gas-permeable film forming the container 30 is folded multiple times. The inner faces of the gas-permeable film in this labyrinth structure portion with a labyrinth structure may be caused to adhere to each other in an easily peelable manner, for example, using an easy peel seal or the like. The separating portion 31 (the labyrinth structure portion) is held by, for example, a holding member 35 constituted by a clip or the like. When the holding member 35 holds the separating portion 31 of the container 30, the separating portion 31 is pressed from the outside, and maintains a state in which the first container section 32 and the second container section 33 are separated from each other. Thus, the first agent 1 and the second agent 2 can be stably stored for a long period of time before use.

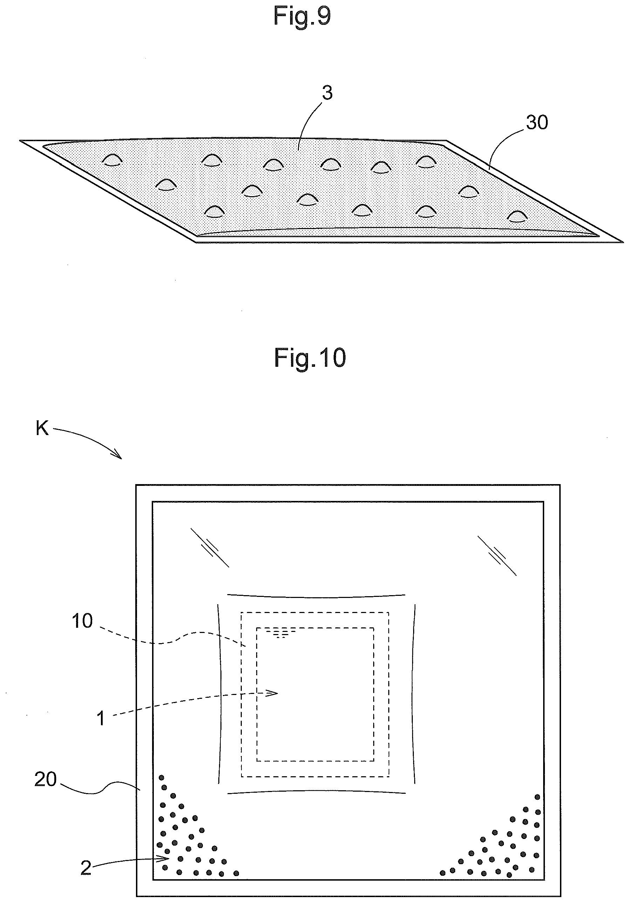

Chlorine dioxide gas can be actually generated using the chlorine dioxide gas generating kit K of this embodiment as follows. That is to say, as shown in FIG. 8, first, the holding member 35 holding the separating portion 31 is manually detached from the container 30. Subsequently, the folded portion is stretched out, so that the labyrinth structure of the separating portion 31 is canceled. Then, the first agent 1 contained in the first container section 32 and the second agent 2 contained in the second container section 33 are mixed with each other inside the container 30, by applying a force from the outside of the container 30. Then, the content is converted into a gel form in the container 30, and chlorine dioxide gas is immediately generated at a stable concentration from the obtained gel composition 3 (see FIG. 9). The container 30 is made of a gas-permeable film, and thus generated chlorine dioxide gas permeates the container 30 and is released into a room. Thus, an antibacterial effect, a deodorant effect, and the like can be provided immediately at the time of use and stably for a long period of time due to the strong oxidizability of chlorine dioxide gas gradually released immediately and at a stable concentration.

In the description above, a configuration may also be employed in which the second agent 2 does not contain the absorbent resin, and only the aqueous chlorite solution, the fast-acting activator, the metal iodide, and the slow-acting activation inhibitor are mixed with each other. In this case, chlorine dioxide gas can be immediately generated at a stable concentration from the obtained liquid composition. Also, in this case, an antibacterial effect, a deodorant effect, and the like can be provided immediately at the time of use and stably for a long period of time due to the strong oxidizability of chlorine dioxide gas gradually released immediately and at a stable concentration.

### Third Embodiment

Hereinafter, a chlorine dioxide gas generating method, a liquid composition, a gel composition, and a chlorine dioxide gas generating kit according to a third embodiment will be described. In this embodiment, the aqueous chlorite solution, the fast-acting activator, the metal iodide, slow-acting activation inhibitor, and the absorbent resin are allocated to the first agent 1 and the second agent 2 of the chlorine dioxide gas generating kit K in a manner different from that of the first and second embodiments. Furthermore, the specific configuration of the container of the chlorine dioxide gas generating kit K is different from that of the first and second embodiments. Hereinafter, the chlorine dioxide gas generating kit K of this embodiment will be described mainly focusing on differences from the first embodiment. Note that constituent elements not specifically described are as in the first embodiment, and thus they are denoted by the same reference numerals, and a description thereof has been omitted.

The chlorine dioxide gas generating kit K of this embodiment includes a first agent 1 containing an aqueous chlorite solution and a metal iodide, and a second agent 2 containing a fast-acting activator, a slow-acting activation inhibitor, and an absorbent resin. As shown in FIG. 10, the first agent 1 is sealed in the first container 10, and the second agent 2 is sealed together with the first container 10 in the second container 20.

The first container 10 is obtained, for example, by sticking plastic films to each other. The first container 10 may be obtained by stacking two plastic films and causing their entire peripheral edge portions to undergo easy peel sealing, or by folding one plastic film in half and causing the peripheral edge portions other than the folded portion to undergo easy peel sealing. In this manner, the first agent 1 is sealed in the sealable and easily breakable first container 10.

The second container 20 is made of a gas-permeable film. The second container 20 may be obtained by stacking two gas-permeable films and causing their entire peripheral edge portions to adhere to each other, or by folding one gas-permeable film in half and causing the peripheral edge portions other than the folded portion to adhere to each other. In this manner, the second agent 2 is sealed together with the first container 10 in the sealable and gas-permeable second container 20.

Chlorine dioxide gas can be actually generated using the chlorine dioxide gas generating kit K of this embodiment as follows. That is to say, the first container 10 is broken inside the second container 20, by applying a force from the outside of the second container 20 to a region in which the first container 10 is located. For example, an easily peelable portion of the first container 10 is peeled away by applying a force from the outside, and the first agent 1 formed as a liquid (an aqueous chlorite solution in which the metal iodide is dissolved) is released from the first container 10. Then, chlorine dioxide gas is immediately generated at a stable concentration from a gel composition obtained by mixing the first agent 1 and the second agent 2. The second container 20 is made of a gas-permeable film, and thus generated chlorine dioxide gas permeates the second container 20 and is released into a room. Thus, an antibacterial effect, a deodorant effect, and the like can be provided immediately at the time of use and stably for a long period of time due to the strong oxidizability of chlorine dioxide gas gradually released immediately and at a stable concentration.

In the description above, a configuration may also be employed in which the second agent 2 does not contain the absorbent resin, and only the aqueous chlorite solution, the fast-acting activator, the metal iodide, and the slow-acting activation inhibitor are mixed with each other. In this case, chlorine dioxide gas can be immediately generated at a stable concentration from the obtained liquid composition. Also, in this case, an antibacterial effect, a deodorant effect, and the like can be provided immediately at the time of use and stably for a long period of time due to the strong oxidizability of chlorine dioxide gas gradually released immediately and at a stable concentration.

Hereinafter, the present invention will be described in more detail by way of examples.

### Example 1

First, 100 g of 4000 ppm aqueous sodium chlorite solution was prepared by dissolving sodium chlorite in pure water. Then, 1.71 g of 7.3% hydrochloric acid serving as an activator, 0.15 g of potassium iodide, and 0.11 g of sodium silicate pentahydrate serving as an activation inhibitor were mixed with the aqueous sodium chlorite solution. The potassium iodide and the sodium silicate pentahydrate were contained in the mixed liquid respectively in proportions in % by mass of 0.37% and 0.27%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Example 2

First, 100 g of 4000 ppm aqueous sodium chlorite solution was prepared by dissolving sodium chlorite in pure water. Then, 1.71 g of 7.3% hydrochloric acid serving as an activator, 0.08 g of potassium iodide, and 0.2 g of sodium silicate pentahydrate serving as an activation inhibitor were mixed with the aqueous sodium chlorite solution. The potassium iodide and the sodium silicate pentahydrate were contained in the mixed liquid respectively in proportions in % by mass of 0.2% and 0.5%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Example 3

First, 100 g of 4000 ppm aqueous sodium chlorite solution was prepared by dissolving sodium chlorite in pure water. Then, 2 g of phosphoric acid serving as an activator, 0.1 g of potassium iodide, and 0.11 g of sodium silicate pentahydrate serving as an activation inhibitor were mixed with the aqueous sodium chlorite solution. The potassium iodide and the sodium silicate pentahydrate were contained in the mixed liquid respectively in proportions in % by mass of 0.25% and 0.27%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Example 4

First, 100 g of 11250 ppm aqueous sodium chlorite solution was prepared by dissolving sodium chlorite in pure water. Then, 1.16 g of phosphoric acid serving as an activator, 0.1 g of potassium iodide, and 0.05 g of sodium silicate pentahydrate serving as an activation inhibitor were mixed with the aqueous sodium chlorite solution. The potassium iodide and the sodium silicate pentahydrate were contained in the mixed liquid respectively in proportions in % by mass of 0.09% and 0.04%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Example 5

First, 100 g of 120000 ppm aqueous sodium chlorite solution was prepared by dissolving sodium chlorite in pure water. Then, 6.3 g of phosphoric acid serving as an activator, 0.1 g of potassium iodide, and 1.19 g of sodium silicate pentahydrate serving as an activation inhibitor were mixed with the aqueous sodium chlorite solution. The potassium iodide and the sodium silicate pentahydrate were contained in the mixed liquid respectively in proportions in % by mass of 0.01% and 0.1%, with respect to 1 L of 1% by mass aqueous chlorite solution (i.e., with respect to 10000 ppm of chlorite). Subsequently, the mixed liquid was stored in a non-sealed state at room temperature, and the pH of the mixed liquid and the concentration of generated chlorine dioxide gas were measured in an open system.

### Example 6

First, 100 g of 120000 ppm aqueous sodium chlorite solution was prepared by dissolving sodium chlorite in pure water. Then, 6.3 g of phosphoric acid serving as an activator, 0.25 g of potassium iodide, and 1.19 g of sodium silicate pentahydrate serving as an activation inhibitor were mixed with the aqueous sodium chlorite solution. The potassium iodide and the sodium silicate pentahydrate were contained in the mixed liquid respectively in proportions in % by mass of 0.02% and 0.1%, with respect to 1 L of 1% by mass aqueous chlorite solution (i.e., with respect to 10000 ppm of chlorite). Subsequently, the mixed liquid was stored in a non-sealed state at room temperature, and the pH of the mixed liquid and the concentration of generated chlorine dioxide gas were measured in an open system.

### Example 7

First, 100 g of 120000 ppm aqueous sodium chlorite solution was prepared by dissolving sodium chlorite in pure water. Then, 6.3 g of phosphoric acid serving as an activator, 0.5 g of potassium iodide, and 1.19 g of sodium silicate pentahydrate serving as an activation inhibitor were mixed with the aqueous sodium chlorite solution. The potassium iodide and the sodium silicate pentahydrate were contained in the mixed liquid respectively in proportions in % by mass of 0.04% and 0.1%, with respect to 1 L of 1% by mass aqueous chlorite solution (i.e., with respect to 10000 ppm of chlorite). Subsequently, the mixed liquid was stored in a non-sealed state at room temperature, and the pH of the mixed liquid and the concentration of generated chlorine dioxide gas were measured in an open system.

### Example 8

First, 100 g of 120000 ppm aqueous sodium chlorite solution was prepared by dissolving sodium chlorite in pure water. Then, 6.3 g of phosphoric acid serving as an activator, 1 g of potassium iodide, and 1.19 g of sodium silicate pentahydrate serving as an activation inhibitor were mixed with the aqueous sodium chlorite solution. The potassium iodide and the sodium silicate pentahydrate were contained in the mixed liquid respectively in proportions in % by mass of 0.08% and 0.1%, with respect to 1 L of 1% by mass aqueous chlorite solution (i.e., with respect to 10000 ppm of chlorite). Subsequently, the mixed liquid was stored in a non-sealed state at room temperature, and the pH of the mixed liquid and the concentration of generated chlorine dioxide gas were measured in an open system.

### Comparative Example 1

First, 100 g of 4000 ppm aqueous sodium chlorite solution was prepared by dissolving sodium chlorite in pure water. Then, 1.71 g of 7.3% hydrochloric acid serving as an activator and 0.11 g of sodium silicate pentahydrate serving as an activation inhibitor were mixed with the aqueous sodium chlorite solution. The sodium silicate pentahydrate was contained in the mixed liquid in a proportion in % by mass of 0.27%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Comparative Example 2

First, 100 g of 4000 ppm aqueous sodium chlorite solution was prepared by dissolving sodium chlorite in pure water. Then, 1.71 g of 7.3% hydrochloric acid serving as an activator and 0.15 g of potassium iodide were mixed with the aqueous sodium chlorite solution. The potassium iodide was contained in the mixed liquid in a proportion in % by mass of 0.37%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Comparative Example 3

First, 100 g of 4000 ppm aqueous sodium chlorite solution was prepared by dissolving sodium chlorite in pure water. Then, 2 g of phosphoric acid serving as an activator and 0.11 g of sodium silicate pentahydrate serving as an activation inhibitor were mixed with the aqueous sodium chlorite solution. The sodium silicate pentahydrate was contained in the mixed liquid in a proportion in % by mass of 0.27%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Comparative Example 4

First, 100 g of 4000 ppm aqueous sodium chlorite solution was prepared by dissolving sodium chlorite in pure water. Then, 2 g of phosphoric acid serving as an activator and 0.1 g of potassium iodide were mixed with the aqueous sodium chlorite solution. The potassium iodide was contained in the mixed liquid in a proportion in % by mass of 0.25%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Comparative Example 5

First, 100 g of 11250 ppm aqueous sodium chlorite solution was prepared by dissolving sodium chlorite in pure water. Then, 1.16 g of phosphoric acid serving as an activator and 0.05 g of sodium silicate pentahydrate serving as an activation inhibitor were mixed with the aqueous sodium chlorite solution. The sodium silicate pentahydrate was contained in the mixed liquid in a proportion in % by mass of 0.04%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Comparative Example 6

First, 100 g of 11250 ppm aqueous sodium chlorite solution was prepared by dissolving sodium chlorite in pure water. Then, 1.16 g of phosphoric acid serving as an activator and 0.1 g of potassium iodide were mixed with the aqueous sodium chlorite solution. The potassium iodide was contained in the mixed liquid in a proportion in % by mass of 0.09%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

### Comparative Example 7

First, 100 g of 11250 ppm aqueous sodium chlorite solution was prepared by dissolving sodium chlorite in pure water. Then, 3.01 g of 7.3% hydrochloric acid serving as an activator, 0.5 g of potassium iodide, and 2 g of sodium silicate pentahydrate serving as an activation inhibitor were mixed with the aqueous sodium chlorite solution. The potassium iodide and the sodium silicate pentahydrate were contained in the mixed liquid respectively in proportions in % by mass of 0.44% and 1.78%, with respect to 1 L of 1% by mass aqueous chlorite solution (i.e., with respect to 10000 ppm of chlorite). Subsequently, the mixed liquid was stored in a sealed state at room temperature, and the pH of the mixed liquid and the concentration of generated chlorine dioxide gas were measured in a closed system.

**Table 1**

| | NaClO₂ (ppm) | Hydrochloric acid (g) | Phosphoric acid (g) | KI (g) | Na (g) | KI content (1/10000ppm) | Na-silicate content (1/10000ppm) |
|---|---|---|---|---|---|---|---|
| Ex. 1 | 4000 | 1.71 | - | 0.15 | 0.11 | 0.37% | 0.27% |
| Ex. 2 | 4000 | 1.71 | - | 0.08 | 0.20 | 0.20% | 0.50% |
| Ex. 3 | 4000 | - | 2.00 | 0.10 | 0.11 | 0.25% | 0.27% |
| Ex. 4 | 11250 | - | 1.16 | 0.10 | 0.05 | 0.09% | 0.04% |
| Ex. 5 | 120000 | - | 6.30 | 0.10 | 1.19 | 0.01% | 0.10% |
| Ex. 6 | 120000 | - | 6.30 | 0.25 | 1.19 | 0.02% | 0.10% |
| Ex. 7 | 120000 | - | 6.30 | 0.50 | 1.19 | 0.04% | 0.10% |
| Ex. 8 | 120000 | - | 6.30 | 1.00 | 1.19 | 0.08% | 0.10% |
| Com.Ex. 1 | 4000 | 1.71 | - | - | 0.11 | - | 0.27% |
| Com.Ex. 2 | 4000 | 1.71 | - | 0.15 | - | 0.37% | - |
| Com.Ex. 3 | 4000 | - | 2.00 | - | 0.11 | - | 0.27% |
| Com.Ex. 4 | 4000 | - | 2.00 | 0.10 | - | 0.25% | - |
| Com.Ex. 5 | 11250 | - | 1.16 | - | 0.05 | - | 0.04% |
| Com.Ex. 6 | 11250 | - | 1.16 | 0.10 | - | 0.09% | - |
| Com.Ex. 7 | 11250 | 3.01 | - | 0.50 | 2.00 | 0.44% | 1.78% |

Measurement results were as follows.

**Table 2**

| | Elapsed time | 1 min | 10 min | 30 min | 24 hr | 7 d |
|---|---|---|---|---|---|---|
| Ex. 1 | pH | 2.8 | 2.8 | 2.8 | 2.9 | 2.9 |
| | ClO₂ (ppm) | 1028 | 1032 | 1045 | 998 | 1119 |
| Ex. 1 | pH | 3.0 | 3.0 | 3.0 | 3.5 | 3.6 |
| | ClO₂ (ppm) | 581 | 554 | 534 | 696 | 517 |
| Com. Ex. 1 | pH | 2.5 | 2.5 | 2.5 | 2.8 | 2.8 |
| | ClO₂ (ppm) | 67 | 111 | 178 | 897 | 894 |
| Com. Ex. 1 | pH | 2.4 | 2.4 | 2.4 | 2.4 | 2.3 |
| | ClO₂ (ppm) | 1354 | 1418 | 1482 | 1468 | 732 |

In Comparative Example 1 in which a potassium iodide was not mixed, the generation of chlorine dioxide gas in the early stage (especially within 30 minutes) after mixing was slow, and, in Comparative Example 2 in which sodium silicate pentahydrate was not mixed, the concentration of chlorine dioxide gas after a long period of time had elapsed (specifically, after 7 days) was unstable. On the other hand, in Examples 1 and 2 in which both a potassium iodide and sodium silicate pentahydrate were mixed, it was seen that chlorine dioxide gas was released at a stable concentration over a long period of time from the early stage after mixing.

**Table 3**

| | Elapsed time | 1 min | 10 min | 30 min | 24 hr | 7 d |
|---|---|---|---|---|---|---|
| Ex. 3 | pH | 5.9 | 5.9 | 6.0 | 6.0 | 6.0 |
| | ClO₂ (ppm) | 118 | 128 | 134 | 148 | 134 |
| Com.Ex. 3 | pH | 5.9 | 5.9 | 5.9 | 5.9 | 5.9 |
| | ClO₂ (ppm) | 0 | 3 | 5 | 10 | 17 |
| Com.Ex. 4 | pH | 5.4 | 5.4 | 5.4 | 5.5 | 5.4 |
| | ClO₂ (ppm) | 242 | 242 | 239 | 186 | 128 |

In Comparative Example 3 in which a potassium iodide was not mixed, generation of chlorine dioxide gas was slow, and, in Comparative Example 4 in which sodium silicate pentahydrate was not mixed, the concentration of chlorine dioxide gas after a long period of time had elapsed (specifically, after 7 days) was unstable. On the other hand, in Example 3 in which both a potassium iodide and sodium silicate pentahydrate were mixed, it is seen that, even when a weak acid was used as an activator, chlorine dioxide gas was promptly released from the early stage (substantially within 10 minutes) after mixing. Furthermore, it was seen that chlorine dioxide gas was released at a stable concentration over a long period of time after that early stage.

**Table 4**

| | Elapsed time | 1 min | 10 min | 30 min | 24 hr | 7 d |
|---|---|---|---|---|---|---|
| Ex. 4 | pH | 6.0 | 6.0 | 6.0 | 6.0 | 6.0 |
| | ClO₂ (ppm) | 144 | 138 | 148 | 128 | 158 |
| Com.Ex. 5 | pH | 5.9 | 5.9 | 5.9 | 5.9 | 6.0 |
| | ClO₂ (ppm) | 14 | 23 | 35 | 24 | 67 |
| Com.Ex. 6 | pH | 5.6 | 5.6 | 5.6 | 5.7 | 5.7 |
| | ClO₂ (ppm) | 208 | 201 | 192 | 141 | 138 |
| Com.Ex. 7 | pH | 12.7 | 12.7 | 12.7 | 12.8 | 12.8 |
| | ClO₂ (ppm) | 0 | 0 | 0 | 0 | 0 |

In Comparative Example 5 in which a potassium iodide was not mixed, generation of chlorine dioxide gas was slow, and, in Comparative Example 6 in which sodium silicate pentahydrate was not mixed, the concentration of chlorine dioxide gas after a predetermined period of time had elapsed (after 24 hours) was unstable. On the other hand, in Example 4 in which both a potassium iodide and sodium silicate pentahydrate were mixed, it was seen that, even when a weak acid was used as an activator, chlorine dioxide gas was promptly released from the very early stage (within 1 minute) after mixing. Furthermore, it was seen that chlorine dioxide gas was released at a stable concentration over a long period of time after that very early stage.

Furthermore, in Comparative Example 7 in which both a potassium iodide and sodium silicate pentahydrate were mixed but the amounts thereof added were excessive, generation of chlorine dioxide gas itself was not seen. On the other hand, referring to Examples 1 to 4, it was seen that, when the potassium iodide and the sodium silicate pentahydrate were contained respectively in proportions in % by mass of 0.4% or less and 1% or less, with respect to 1 L of 1% by mass aqueous chlorite solution (i.e., with respect to 10000 ppm of chlorite), chlorine dioxide gas was promptly released at a stable concentration.

**Table 5**

| | Elapsed time | 1 min | 10 min | 30 min | 24 hr | 7 d |
|---|---|---|---|---|---|---|
| Ex. 5 | pH | 6.0 | 6.0 | 6.0 | 6.1 | 6.2 |
| | ClO₂ (ppm) | 521 | 511 | 507 | 837 | 1045 |
| Ex. 6 | pH | 6.0 | 6.0 | 6.0 | 6.1 | 6.2 |
| | ClO₂ (ppm) | 709 | 712 | 689 | 954 | 1038 |
| Ex. 7 | pH | 6.1 | 6.1 | 6.1 | 6.2 | 6.2 |
| | ClO₂ (ppm) | 907 | 900 | 860 | 914 | 857 |
| Ex. 8 | pH | 6.1 | 6.1 | 6.1 | 6.1 | 6.1 |
| | ClO₂ (ppm) | 1193 | 1169 | 1068 | 1048 | 827 |

In Examples 5 to 8, it was seen that, substantially, chlorine dioxide gas was promptly released at a stable concentration also in an open system.

In the description above, embodiments (including examples) of the chlorine dioxide gas generating method, the liquid composition, the gel composition, and the chlorine dioxide gas generating kit K were described in detail by way of specific examples, but the scope of the present invention is not limited to the foregoing specific examples and embodiments. The examples and embodiments disclosed in this specification are, in all respects, illustrative and not limiting. Various modifications may be made without departing from the gist of the invention.

For example, a configuration may also be employed in which the first agent 1 contains the aqueous chlorite solution and the slow-acting activation inhibitor, and the second agent 2 contains the fast-acting activator, the metal iodide, and the absorbent resin.

### Description of Reference Signs

- 1: First agent
- 2: Second agent
- 3: Gel composition
- 10: First container
- 11: Container main body
- 12: Sealing cap
- 14: Opening cap
- 15: Opening
- 20: Second container
- 30: Container
- 31: Separating portion
- 32: First container section
- 33: Second container section
- 35: Holding member
- K: Chlorine dioxide gas generating kit

## Claims

1. A chlorine dioxide gas generating method for immediately generating chlorine dioxide gas at a stable concentration from a liquid composition, comprising obtaining the composition by mixing an aqueous chlorite solution, an activator that immediately adjusts a pH of the aqueous chlorite solution, thereby causing the aqueous chlorite solution to generate chlorine dioxide gas, an activation inhibitor that slowly mitigates an action of the activator, and a metal iodide such that the metal iodide is contained in a proportion of 0.4% by mass or less and the activation inhibitor is contained in a proportion of 1% by mass or less with respect to 1 L of 1% by mass aqueous chlorite solution.

2. A chlorine dioxide gas generating method for immediately generating chlorine dioxide gas at a stable concentration from a gel composition, comprising obtaining the composition by mixing an aqueous chlorite solution, an activator that immediately adjusts a pH of the aqueous chlorite solution, thereby causing the aqueous chlorite solution to generate chlorine dioxide gas, an activation inhibitor that slowly mitigates an action of the activator, a metal iodide, and an absorbent resin such that the metal iodide is contained in a proportion of 0.4% by mass or less and the activation inhibitor is contained in a proportion of 1% by mass or less with respect to 1 L of 1% by mass aqueous chlorite solution.

3. A liquid composition for immediately generating chlorine dioxide gas at a stable concentration, comprising an aqueous chlorite solution, an activator that immediately adjusts a pH of the aqueous chlorite solution, thereby causing the aqueous chlorite solution to generate chlorine dioxide gas, an activation inhibitor that slowly mitigates an action of the activator, and a metal iodide such that the metal iodide is contained in a proportion of 0.4% by mass or less and the activation inhibitor is contained in a proportion of 1% by mass or less with respect to 1 L of 1% by mass aqueous chlorite solution.

4. A gel composition for immediately generating chlorine dioxide gas at a stable concentration, comprising an aqueous chlorite solution, an activator that immediately adjusts a pH of the aqueous chlorite solution, thereby causing the aqueous chlorite solution to generate chlorine dioxide gas, an activation inhibitor that slowly mitigates an action of the activator, a metal iodide, and an absorbent resin such that the metal iodide is contained in a proportion of 0.4% by mass or less and the activation inhibitor is contained in a proportion of 1% by mass or less with respect to 1 L of 1% by mass aqueous chlorite solution.

5. A chlorine dioxide gas generating kit for immediately generating chlorine dioxide gas at a stable concentration from a liquid composition, comprising:
a first agent containing an aqueous chlorite solution; and
a second agent containing an activator that immediately adjusts a pH of the aqueous chlorite solution, thereby causing the aqueous chlorite solution to generate chlorine dioxide gas,
wherein each of an activation inhibitor that slowly mitigates an action of the activator, and a metal iodide is contained in either the first agent or the second agent,
0.4% by mass or less of the metal iodide and 1% by mass or less of the activation inhibitor are contained with respect to 1 L of 1% by mass aqueous chlorite solution, and
the composition is obtained by mixing the first agent and the second agent.

6. A chlorine dioxide gas generating kit for immediately generating chlorine dioxide gas at a stable concentration from a gel composition, comprising:
a first agent containing an aqueous chlorite solution; and
a second agent containing an activator that immediately adjusts a pH of the aqueous chlorite solution, thereby causing the aqueous chlorite solution to generate chlorine dioxide gas, and an absorbent resin,
wherein each of an activation inhibitor that slowly mitigates an action of the activator, and a metal iodide is contained in either the first agent or the second agent,
0.4% by mass or less of the metal iodide and 1% by mass or less of the activation inhibitor are contained with respect to 1 L of 1% by mass aqueous chlorite solution, and
the composition is obtained by mixing the first agent and the second agent.

7. The chlorine dioxide gas generating kit according to claim 5 or 6, wherein 0.03% by mass or more and 0.3% by mass or less of the activation inhibitor is contained with respect to 1 L of 1% by mass aqueous chlorite solution.

8. The chlorine dioxide gas generating kit according to any one of claims 5 to 7, wherein 0.01% by mass or more and 0.4% by mass or less of the metal iodide is contained with respect to 1 L of 1% by mass aqueous chlorite solution.

9. The chlorine dioxide gas generating kit according to any one of claims 5 to 8, wherein a mass ratio between the activation inhibitor and the metal iodide is 3 : 1 to 1 : 3.

10. The chlorine dioxide gas generating kit according to any one of claims 5 to 9, wherein the activation inhibitor is an alkali metal silicate or an alkaline-earth metal silicate.

11. The chlorine dioxide gas generating kit according to claim 10, wherein the activation inhibitor is a sodium silicate.

12. The chlorine dioxide gas generating kit according to any one of claims 5 to 11, wherein the metal iodide is a potassium iodide.

13. The chlorine dioxide gas generating kit according to any one of claims 5 to 12, wherein the activator is an inorganic acid or an organic acid, or a salt thereof.

14. The chlorine dioxide gas generating kit according to any one of claims 5 to 13, wherein the first agent is sealed in a sealable first container, and the second agent is sealed in a sealable second container that is different from the first container.

15. The chlorine dioxide gas generating kit according to any one of claims 5 to 13, wherein the first agent and the second agent are sealed in the same sealable container in a state of being separated from each other by a separating portion that can be manually deactivated.

16. The chlorine dioxide gas generating kit according to claim 15, wherein the separating portion is constituted by a labyrinth structure portion.

17. The chlorine dioxide gas generating kit according to any one of claims 5 to 13, wherein the first agent is sealed in a sealable and easily breakable first container, and the second agent is sealed together with the first container in a sealable second container.
